# EUROPEAN PATENT SPECIFICATION

(11) **EP 4 277 552 B1**
(45) Date of publication and mention of the grant of the patent: **02.12.2026**
(21) Application number: 22740025.6
(22) Date of filing: 13.01.2022
(51) Int. Cl.: A61B 17/58, A61B 17/68, A61B 17/84, A61F 2/28, A61F 2/30, A61F 2/32

(54) **ORTHOPEDIC IMPLANT**
ORTHOPÄDISCHES IMPLANTAT
IMPLANT ORTHOPÉDIQUE

(30) Priority: 13.01.2021 US 202163136911 P
(43) Date of publication of application: 22.11.2023
(73) Proprietor: Bone Solutions, Inc., Colleyville, TX 76034 (US)
(72) Inventor: MORRIS, Frankie, L., Colleyville, TX 76034 (US)
(74) Representative: Zwicker, Jörk
(86) International application number: PCT/US2022/012225
(87) International publication number: WO 2022/155275

(56) References cited:
- US-A- 6 165 219
- US-A1- 2002 123 752
- US-A1- 2007 050 031
- US-A1- 2007 050 031
- US-A1- 2011 060 373
- US-A1- 2013 296 953
- US-A1- 2016 058 568
- US-A1- 2020 022 817

## Description

### TECHNICAL FIELD

The present disclosure relates to an orthopedic implant and methods of use. Embodiments of the orthopedic implant are used for fixing various devices to or in a bone of a patient.

### BACKGROUND

Unless otherwise indicated herein, the materials described in this section are not admitted to be prior art to the claims in this application.

Many procedures in the field of orthopedics require the use of screws, anchors, pins, or other such fixation devices. In one example, such fixation devices may be used to attach soft tissue such as ligaments, tendons, or muscles to a surface from which the soft tissue has become detached. For example, the rotator cuff may be reattached to the humeral head during a shoulder repair. As another example, fixation devices may be used in the reconstruction of the anterior cruciate ligament (ACL) to secure a substitute ligament to the tibia and the femur. Fixation devices may also be used to secure soft tissue to supplementary attachment sites for reinforcement. For example, in urological applications, fixation devices may be used in bladder neck suspension procedures to attach a portion of the bladder to an adjacent bone surface. Such soft tissue attachments may be done during either open or closed surgical procedures, the latter being generally referred to as arthroscopic or endoscopic surgery. The terms "arthroscopic" and "endoscopic" may be used interchangeably herein and are intended to encompass arthroscopic, endoscopic, laparoscopic, hysteroscopic or any other similar surgical procedures performed with elongated instruments inserted through small openings in the body. Many other potential uses of various fixation devices are possible as well.

Such fixation devices make take a variety of forms. Typically, metal screws (often made of titanium) are used for this purpose and are the preferred choice for surgeons as they cause minor inflammatory response. However, the use of metal screws often requires a second surgical intervention to remove the screw after healing. Also, because mechanical stresses are borne to a large part by rigid metal screws, the surrounding bone does not carry sufficient load during and after the healing process to produce a biologically strong structure. In some cases, this can cause rise to post-operative complications a number of years after implantation.

Synthetic polymer screws are currently available and are an alternative choice to metal screws. As the polymer is degraded and absorbed by the body during the months following surgery, the screw site is replaced by biological tissue and so the biomechanical stresses are transferred from the implant or screw to the newly-formed tissue produced during the healing process. Such synthetic polymer screws are typically heavy in poly-lactic acid and tri-calcium phosphate, the combination of which take a long time to be absorbed into the body. The use of different types of materials such as magnesium-based, iron-based, and zinc-based alloys may help to speed up absorption rates with their known biocompatibility and material properties. The problem with such materials is that in their purist form, these materials have shown to have high corrosion effects during in-vitro and in vivo experiments. US 2016/058568 A1, for example, discloses bioactive spinal implants and a method of manufacture thereof.
US 2007/050031 A1, for example, discloses an intervertebral implant includes an implant body having superior and inferior sides, medial and lateral sides, and anterior and posterior ends.

### SUMMARY

In view of the foregoing, the inventors recognized that an improved orthopedic implant would be desirable. The present invention provides such a device and is defined in claim 1.

In a first aspect, the present disclosure provides an orthopedic implant. The orthopedic implant includes an elongated member having a first end and a second end opposite the first end. The elongated member is tapered at the second end such that a width of the second end is less than a width of the first end. The orthopedic implant also includes a first channel positioned on a first side of the elongated member and extending from the first end to the second end. The orthopedic implant also includes a second channel positioned on a second side of the elongated member and extending from the first end to the second end. The orthopedic implant also includes one or more through holes connecting the first channel to the second channel. A plurality of threads is provided on at least a portion of an exterior of the elongated member.

The invention may be used in a method for securing an orthopedic implant to a bone, the method comprising: (a) providing the orthopedic implant of the first aspect, (b) forming a tunnel in the bone, (c) inserting the second end of the elongated member of the orthopedic implant into the tunnel in the bone, and (d) applying an uncured osteostimulative material to one or more of the first channel and the second channel of the elongated member.

These as well as other aspects, advantages, and alternatives, will become apparent to those of ordinary skill in the art by reading the following detailed description, with reference where appropriate to the accompanying drawings.

### BRIEF DESCRIPTION OF THE DRAWINGS

FIGURE 1 illustrates a top view of an orthopedic implant.
FIGURE 2 illustrates a side view of the orthopedic implant of Figure 1. [0013] FIGURE 3 illustrates a perspective view of the orthopedic implant of Figure 1.
FIGURE 4 illustrates a perspective view of another orthopedic implant.
FIGURE 5 illustrates a perspective view of another orthopedic implant.

### DETAILED DESCRIPTION

Example methods and systems are described herein. It should be understood that the words "example," "exemplary," and "illustrative" are used herein to mean "serving as an example, instance, or illustration." Any embodiment or feature described herein as being an "example," being "exemplary," or being "illustrative" is not necessarily to be construed as preferred or advantageous over other embodiments or features. The exemplary embodiments described herein are not meant to be limiting. It will be readily understood that the aspects of the present disclosure, as generally described herein and illustrated in the figures, can be arranged, substituted, combined, separated, and designed in a wide variety of different configurations, all of which are explicitly contemplated herein.

Furthermore, the particular arrangements shown in the Figures should not be viewed as limiting. It should be understood that other embodiments may include more or less of each element shown in a given Figure. Further, some of the illustrated elements may be combined or omitted. Yet further, an exemplary embodiment may include elements that are not illustrated in the Figures.

As used herein, with respect to measurements, "about" means +/- 5%.

As used herein, "osteostimulative" refers to the ability of a material to improve healing of bone injuries or defects.

As used herein, "osteoconductive" refers to the ability of a material to serve as a scaffold for viable bone growth and healing.

As used herein, "osteoinductive" refers to the capacity of a material to stimulate or induce bone growth.

As used herein, "biocompatible" refers to a material that elicits no significant undesirable response when inserted into a recipient (*e.g.,* a mammalian, including human, recipient).

As used herein, "resorbable" refers to a material's ability to be absorbed in-vivo through bodily processes. The absorbed material may turn into bone in the patient's body.

The present disclosure provides an orthopedic implant suitable for use in orthopedic surgery. The orthopedic implant described herein may be used in conjunction with cement or bone void fillers. In one example, the use of a polymer comprising poly-lactic acid and magnesium phosphate further allows the orthopedic implant to be absorbed in-vivo, producing increased fixation strength and faster absorption into the body.

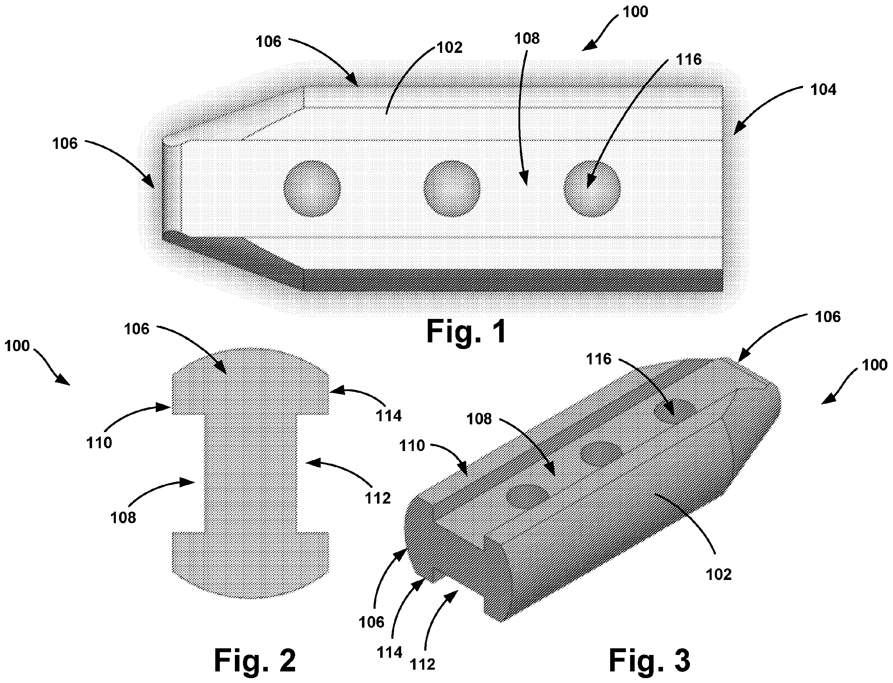

With reference to the Figures, Figures 1-3 illustrate an exemplary orthopedic implant 100. As shown in Figures 1-3, the orthopedic implant 100 includes an elongated member 102 having a first end 104 and a second end 106 opposite the first end 104. The elongated member 102 is tapered at the second end 106 such that a width of the second end 106 is less than a width of the first end 104. The orthopedic implant 100 further includes a first channel 108 positioned on a first side 110 of the elongated member 102 and extending from the first end 104 to the second end 106. The orthopedic implant 100 further includes a second channel 112 positioned on a second side 114 of the elongated member 102 and extending from the first end 104 to the second end 106. The orthopedic implant 100 further includes one or more through holes 116 connecting the first channel 108 to the second channel 112.

As shown in Figures 1-3, the orthopedic implant 100 may include a first portion where the first side 110 and the second side 114 are a fixed distance apart, and a second portion that is tapered. The first side 110 may have a radius of curvature for at least the first portion, and the second side 114 may have the radius of curvature for at least the first portion.

As shown in Figure 1, in one example the one or more through holes 116 comprises a plurality of through holes. The first channel 108 is recessed in the first side 110 of the elongated member 102, and the second channel 112 is recessed in the second side 114 of the elongated member 102.

In one example, as shown in Figures 1-3, the exterior surface of the elongated member is unthreaded. In such an example, which does not form part of the present invention, the first end 104 may be substantially flat, such that it is configured to be inserted into a structure via a hammer or other similar tool.

According to the invention, at least a portion of the exterior surface of the elongated member includes a plurality of threads. In such an example, the orthopedic implant 100 may also a drive socket positioned at the first end 104 of the elongated member 102. Such a drive socket may comprise a recessed cutout in the first end 104 of the elongated member 102. In one example, the orthopedic implant 100 also includes a head coupled to the first end 104 of the elongated member 102. The head may have a diameter greater than a diameter of the elongated member 102, thereby providing a stopping point for the orthopedic implant 100 as it is inserted into a structure when in use. The elongated member 102 may be integral to the head such that they are formed unitarily, or the elongated member 102 may be coupled separately to the head. In such an example, the drive socket may be integral to the head, such that the drive socket comprises a recessed cutout in the head.

Such a drive socket is designed to interact with a suitable torque-transmitting insertion device, such as an implant driver, and thereby allow transmission of the requisite amount of torque needed to drive the implant into the prepared socket. For example, the drive socket may be a polygonal recess in the first end 104 of the elongated member 102 while the torque-transmitting feature characterizing the distal end of the driver is a corresponding polygonal protrusion (such as found on the conventional hex key or "Allen" key). In another embodiment, the drive socket may be one or more axially extending slots recessed in the first end 104 of the elongated member 102 while the driver is a slotted, flat blade or crosshead ("Phillips head") screwdriver. However, other embodiments will be readily apparent to the skilled artisan. Moreover, it will be readily understood by the skilled artisan that the position of the respective coordinating elements (e.g., recessed slots and grooves that mate with assorted projecting protrusions, protuberances, tabs and splines) may be exchanged and/or reversed as needed.

In one example, the elongated member 102 further includes a bioactive therapeutic agent for achieving further enhanced bone fusion and ingrowth. In one example, the bioactive therapeutic agent is in the form of a surface coating on the exterior surface of the elongated member 102. In another example, the orthopedic implant 100 may be incorporated into a plurality of pores in the material from which the orthopedic implant 100 is formed. Such bioactive therapeutic agents may include natural or synthetic therapeutic agents such as bone morphogenic proteins (BMPs), growth factors, bone marrow aspirate, stem cells, progenitor cells, antibiotics, amikacin, butirosin, dideoxykanamycin, fortimycin, gentamycin, kanamycin, lividomycin, neomycin, netilmicin, ribostamycin, sagamycin, seldomycin and epimers thereof, sisomycin, sorbistin, spectinomycin and tobramycin, or other osteoconductive, osteoinductive, osteogenic, bio-active, or any other fusion enhancing material or beneficial therapeutic agent.

The elongated member 102 may take a variety of forms. In one example, the elongated member 102 of the orthopedic implant 100 may comprise titanium, polyetheretherketone (PEEK), polyurethane, bone, or combinations thereof.

In another example, the entirety of the orthopedic implant 100 is made from a polymer including poly-lactic acid and either magnesium phosphate or potassium phosphate. As used herein, "poly-lactic acid" or polylactide (PLA) is a biodegradable and bioactive thermoplastic aliphatic polyester derived from renewable resources, and may take a variety of forms including, but not limited to, poly-L-lactide (PLLA), poly-D-lactide (PDLA), and poly(L-lactide-co-D,L-lactide) (PLDLLA). As used herein, "magnesium phosphate" is a general term for salts of magnesium and phosphate appearing in several forms and several hydrates including, but not limited to, monomagnesium phosphate ((Mg(H₂PO₄)₂)·xH₂O), dimagnesium phosphate ((MgHPO₄)·xH₂O), and trimagnesium phosphate ((Mg₃(PO₄)₂)·xH₂O). As used herein, "calcium phosphate" is a family of materials and minerals containing calcium ions (Ca²⁺) together with inorganic phosphate anions and appearing in a variety of forms including, but not limited to, monocalcium phosphate, dicalcium phosphate , tricalcium phosphate, octacalcium phosphate, amorphous calcium phosphate, dicalcium diphosphate, calcium triphosphate, hydroxyapatite, apatite, and tetracalcium phosphate.

Making the entirety of the orthopedic implant 100 from a polymer including poly-lactic acid and either magnesium phosphate or potassium phosphate has a number of advantages. In particular, such a material allows the orthopedic implant 100 to be absorbed in-vivo, producing increased fixation strength and faster absorption into the body. In contrast to traditional metal alloy bone screws, there is no need to remove the orthopedic implant 100 after a certain period of time because the material of the orthopedic implant 100 enables bone to actually replace the structure of the orthopedic implant 100. As such, there is no void that is left behind after the orthopedic implant 100 is absorbed in-vivo. Instead, the orthopedic implant 100 is replaced with bone structure grown naturally in the body and the resulting fixation strength is very strong. As such, the orthopedic implant 100 may be completely resorbable.

The resultant orthopedic implant 100 exhibits relatively high mechanical strength for load bearing support, while additionally and desirably providing high osteoconductive and osteoinductive properties to achieve enhanced bone ingrowth and fusion. In use, the polymer including poly-lactic acid and either magnesium phosphate or potassium phosphate that makes up the orthopedic implant 100 will induce bone growth into the orthopedic implant 100 and be resorbed. The orthopedic implant 100 is eventually replaced by bone in the body, thereby firmly securing the component to which the orthopedic implant 100 (e.g., a substitute ligament in an ACL reconstruction surgery or an existing rotator cuff in a rotator cuff reattachment surgery) is connected to the bone structure the body.

In yet another example, at least a portion of an exterior surface of the elongated member 102 includes a cured osteostimulative material. Such an osteostimulative material may take a variety of forms. The osteostimulative material may allow for in-situ (i.e., in vivo) attachment of biological structures to each other and to manmade structures. The osteostimulative material may also facilitate the repair of bone, ligaments, tendons and adjacent structures. The osteostimulative material may also provide a bone substitute for surgical repair. The formulation of the osteostimulative material is usable at numerous temperatures, pH ranges, humidity levels, and pressures. However, the formulation can be designed to be utilized at all physiological temperatures, pH ranges, and fluid concentrations. The osteostimulative material typically is, but not necessarily, injectable before curing and can exhibit neutral pH after setting. It may be absorbed by the host over a period of time.

In one particular example, the cured osteostimulative material comprises KH₂PO₄ in an amount between about 20-70 dry weight percent, Magnesium oxide (MgO) in an amount between 10-50 dry weight percent, a calcium containing compound, a poly-lactic acid, and either magnesium phosphate or potassium phosphate. The cured osteostimulative material may have both osteoconductive and osteoinductive properties. In addition, the cured osteostimulative material may be bioresorbable. A thickness of the cured osteostimulative material on the exterior surface of the elongated member 102 may range from about 200 µm to about 50 mm. In some examples, the cured osteostimulative material does not cover the entirety of the exterior surface of the elongated member 102 such that there are areas of bare titanium polyetheretherketone (PEEK), polyurethane, and/or bone. In another example, as discussed above, the exterior surface of the elongated member 102 may include a plurality of threads or a plurality of grooves. In such an example, the cured osteostimulative material is positioned in one or more of the plurality of grooves. In another example, the entirety of the elongated member 102 comprises the cured osteostimualtive material.

In accordance with a further aspect of the invention, the orthopedic implant 100 may additionally carry one or more bioactive therapeutic agents for achieving further enhanced bone fusion and ingrowth. Such bioactive therapeutic agents may include natural or synthetic therapeutic agents such as bone morphogenic proteins (BMPs), growth factors, bone marrow aspirate, stem cells, progenitor cells, antibiotics, or other osteoconductive, osteoinductive, osteogenic, bio-active, or any other fusion enhancing material or beneficial therapeutic agent. In another example, the bioactive therapeutic agent comprises one of amikacin, butirosin, dideoxykanamycin, fortimycin, gentamycin, kanamycin, lividomycin, neomycin, netilmicin, ribostamycin, sagamycin, seldomycin and epimers thereof, sisomycin, sorbistin, spectinomycin and tobramycin.

The resultant orthopedic implant 100 exhibits relatively high mechanical strength for load bearing support, while additionally and desirably providing high osteoconductive and osteoinductive properties to achieve enhanced bone ingrowth and fusion. In use, the cured osteostimulative material positioned on the exterior surface of the elongated member 102 of the orthopedic implant 100 will induce bone growth into the orthopedic implant 100 and be resorbed. The osteostimulative material is eventually replaced by bone, thereby more firmly embedding the orthopedic implant 100 in the body.

The osteostimulative material is particularly useful in situations (such as plastic surgery) when the use of metallic fasteners and other non-bioabsorbable materials are to be assiduously avoided. The osteostimulative material also is useful when a certain amount of expansion or swelling is to be expected after surgery, e.g., in skull surgeries. It is a good platform for bone-formation. The osteostimulative material can be also used as an anchoring device or grafting material.

Generally, the osteostimulative material is derived from the hydrated mixture which comprises: (a) KH₂PO₄ in an amount between about 20-70 dry weight percent, (b) MgO in an amount between 10-50 dry weight percent, (c) a calcium containing compound, (d) a sugar. In one particular example, the calcium containing compound is Ca₅(PO₄)₃OH.

Non-limiting exemplary formulations of the osteostimulative material include the following:

### Formulation I *

| | |
|---|---|
| Potassium phosphate (*i.e.,* KH₂PO₄) | 61% |
| MgO (calcined) | 31% |
| Ca₁₀(PO₄)₆(OH)₂ | 4% |
| Sucrose C₁₂H₂₂O₁₁(powder) | 4% |

| | |
|---|---|
| * All values are weight percentages | |

Water is added up to about 40 weight percent of the dry formulation, preferably between about 20-35 weight percent, more preferably between 22-25 weight percent.

### Formulation II *

| | |
|---|---|
| KH₂PO₄ | 54% |
| MgO (calcined) | 33% |
| Ca₁₀(PO₄)₆(OH)₂ | 9% |
| Sucrose C₁₂H₂₂O₁₁(powder) | 4% |

| | |
|---|---|
| * All values are weight percentages | |

Water is added up to about 40 weight percent of the dry formulation, preferably between about 20-35 weight percent, more preferably between 22-25 weight percent.

### Formulation III *

| | |
|---|---|
| KH₂PO₄ | 44% |
| MgO (calcined) | 44% |
| Calcium-containing compound | 8% (whereby the compound is Ca₁₀(PO₄)₆(OH)₂ or CaSiO₃) |
| Sucrose C₁₂H₂₂O₁₁(powder) | 4% |

| | |
|---|---|
| * All values are weight percentages | |

Water is added up to about 40 weight percent of the dry formulation, preferably between about 20-35 weight percent, more preferably between 22-25 weight percent.

### Formulation IV *

| | |
|---|---|
| KH₂PO₄ | 44% |
| MgO (calcined) | 41% |
| Ca₁₀(PO₄)₆(OH)₂ | 8% |
| Sucrose C₁₂H₂₂O₁₁(powder) | 4% |
| Mono-sodium phosphate (MSP) | 3% |

| | |
|---|---|
| * All values are weight percentages | |

Water is added up to about 40 weight percent of the dry formulation, preferably between about 20-35 weight percent, more preferably between about 28-32 weight percent.

### Formulation V*

| | |
|---|---|
| Potassium phosphate (*i.e.,* KH₂PO₄) | 41% |
| MgO (calcined) | 45% |
| Calcium-containing compound | 9% (whereby the compound is Ca₁₀(PO₄)₆(OH)₂, CaSiO₃ or combinations thereof.) |
| Sucrose C₁₂H₂₂O₁₁(powder) | 1% |

| | |
|---|---|
| *All values are weight percentages | |

Water is added up to about 40 weight percent of the dry formulation, preferably between about 20-35 weight percent, more preferably between 22-25 weight percent.

### Formulation VI*

| | |
|---|---|
| KH₂PO₄ | 45% |
| MgO (calcined) | 45% |
| Ca₁₀(PO₄)₆(OH)₂ | 8% |
| Sucralose | 2% |

| | |
|---|---|
| *All values are weight percentages | |

Water is added up to about 40 weight percent of the dry formulation, preferably between about 20-35 weight percent, more preferably between 22-25 weight percent.

### Formulation VII*

| | |
|---|---|
| KH₂PO₄ | 61% |
| MgO (calcined) | 32% |
| Ca₁₀(PO₄)₆(OH)₂ | 4% |
| Dextrose | 1.5% |
| α-Ca₃(PO₄)₂ | 1.5% |

| | |
|---|---|
| *All values are weight percentages | |

Water is added up to about 40 weight percent of the dry formulation, preferably between about 20-35 weight percent, more preferably between 22-25 weight percent.

### Formulation VIII*

| | |
|---|---|
| KH₂PO₄ | 50% |
| MgO (calcined) | 35% |
| Ca₁₀(PO₄)₆(OH)₂ | 7% |
| β-Ca₃(PO₄)₂ | 3% |
| Dextrose | 5% |

| | |
|---|---|
| *All values are weight percentages | |

Water is added up to about 40 weight percent of the dry formulation, preferably between about 20-35 weight percent, more preferably between 22-25 weight percent.

### Formulation IX*

| | |
|---|---|
| KH₂PO₄ | 54% |
| Phosphoric Acid | 4% |
| Metal oxide | 32% (wherein the metal oxide is MgO, ZrO, FeO or a combination thereof) |
| Ca₁₀(PO₄)₆(OH)₂ | 7% |
| Sucrose | 3% |

| | |
|---|---|
| *All values are weight percentages | |

Water is added up to about 40 weight percent of the dry formulation, preferably between about 20-35 weight percent, more preferably between 22-25 weight percent.

### Formulation X*

| | |
|---|---|
| KH₂PO₄ | 61% |
| Metal oxide | 32% (wherein the metal oxide is MgO, Ca, FeO or a combination thereof) |
| Ca₁₀(PO₄)₆(OH)₂ | 6% |
| Sucrose | 1% |

| | |
|---|---|
| *All values are weight percentages | |

Water is added up to about 40 weight percent of the dry formulation, preferably between about 20-35 weight percent, more preferably between 22-25 weight percent.

### Formulation XI*

| | |
|---|---|
| KH₂PO₄ | 45% |
| MgO (calcined) | 45% |
| Ca₁₀(PO₄)₆(OH)₂ | 10% |

| | |
|---|---|
| *All values are weight percentages | |

Water is added up to about 40 weight percent of the dry formulation, preferably between about 20-35 weight percent, more preferably between 22-25 weight percent.

The above formulations and weight percents are merely exemplary. A range of dry constituents can also be used. For example, a suitable range for the phosphate (*i.e.,* monopotassium phosphate (MKP)) is generally between about 20-70 weight percent, preferably between about 40-65 weight percent. In some situations and/or embodiments it is preferable to use the phosphate at a range between about 40-50 weight percent, while in others it may be preferable to use a range of about 50-65 weight percent.

A suitable range for the metal oxide (*i.e.,* MgO) is generally between about 10-60, preferably between 10-50, and even more preferably between 30-50 weight percent. In some situations and/or embodiments it may be preferable to use between about 35 and 50 weight percent.

Calcium containing compounds can be added in various weight percentages. The calcium containing compound(s) is preferably added at about 1-15 weight percent, more preferably between about 1-10 weight percent. Higher percentages can be employed in certain situations.

Sugars (and/or other carbohydrate containing substances) are generally present at weight percent between 0.5 and 20, preferably about 0.5-10 weight percent of the dry composition. The sugars may comprise one of sugar alcohols, sugar acids, amino sugars, sugar polymers glycosaminoglycans, glycolipids, sugar substitutes and combinations thereof.

Water (or another aqueous solution) can be added in a large range of weight percents generally ranging from about 15-40 weight percent, preferably between about 20-35 weight percent. For example, in certain embodiments of the materials as generally described herein, water or other aqueous solution is added at between about 28-32 weight percent. In other embodiments of the materials as generally described herein, water or other aqueous solution is added at between about 28-32 weight percent. It was found that a saline solution may be used. An exemplary saline solution is a 0.9% saline solution.

For some embodiments (*i.e.,* formula III) it has been found that adding water at a weight percent of about 37 weight percent produces a creamy textured material that is extremely easy to work with, has excellent adhesive properties, and is easily injectable through a syringe.

The noted ranges may vary with the addition of various fillers and other components or for other reasons.

In one embodiment, the weight percent ratio between MKP and MgO is between about 4:1 and 0.5:1. In another it is between approximately 2:1 and 1:1.

Without limiting the invention in any manner, in such an embodiment the inventors surmise that the un-reacted magnesium is at least partly responsible for the in vivo expandability characteristics of the bio-adhesive. Specifically the metal oxide (*i.e*., magnesium oxide) reacts with water and serum in and around the living tissue to yield Mg(OH)₂ and magnesium salts. It has been found that in some embodiments the material generally expands to between 0.15 and 0.20 percent of volume during curing in moisture. The expansion of the material is believed to increase the adhesive characteristics of the material. For example, the disclosed material has been shown to effectively attach soft tissues like ligaments to bone, the expansion of the material improving adhesion through mechanical strength.

Osteostimulative material useful in the present invention can also be found in U.S. Patent No. 6,533,821, U.S. Patent No. 6,787,495, U.S. Patent No. 7,045,476, U.S. Patent No. 9,078,884, U.S. Patent Application Publication No. 2015/0250924, and U.S. Patent Application Publication No. 2015/0314045.

In some examples, one or more components of the orthopedic implant 100 described above is made via an additive manufacturing process using an additive-manufacturing machine, such as stereolithography, multi-jet modeling, inkjet printing, selective laser sintering/melting, and fused filament fabrication, among other possibilities. Additive manufacturing enables one or more components of the orthopedic implant 100 and other physical objects to be created as intraconnected single-piece structure through the use of a layer-upon-layer generation process. Additive manufacturing involves depositing a physical object in one or more selected materials based on a design of the object. For example, additive manufacturing can generate one or more components of the orthopedic implant 100 using a Computer Aided Design (CAD) of the orthopedic implant 100 as instructions. As a result, changes to the design of the orthopedic implant 100 can be immediately carried out in subsequent physical creations of the orthopedic implant 100. This enables the components of the orthopedic implant 100 to be easily adjusted or scaled to fit different types of applications (e.g., for use in various wing sizes). In one particular example, the step of applying the uncured osteostimulative material to the exterior surface of the orthopedic implant 100 comprises performing an additive-manufacturing process to deposit the uncured osteostimulative material on the exterior surface of the orthopedic implant 100.

The layer-upon-layer process utilized in additive manufacturing can deposit one or more components of the orthopedic implant 100 with complex designs that might not be possible for devices assembled with traditional manufacturing. In turn, the design of the orthopedic implant 100 can include aspects that aim to improve overall operation. For example, the design can incorporate physical elements that help redirect stresses in a desired manner that traditionally manufactured devices might not be able to replicate.

Additive manufacturing also enables depositing one or more components of the orthopedic implant 100 in a variety of materials using a multi-material additive-manufacturing process. In such an example, the elongated member 102 may be made from a first material and the uncured osteostimulative material may be made from a second material that is different than the first material. In another example, both the elongated member 102 and the uncured osteostimulative material are made from the same material. Other example material combinations are possible as well. Further, one or more components of the orthopedic implant 100 can have some layers that are created using a first type of material and other layers that are created using a second type of material. In addition, various processes are used in other examples to produce one or more components of the orthopedic implant 100. These processes are included in table 1.

**Table 1**

| | |
|---|---|
| DEP | Direct Energy Deposition |
| DMLS | Direct Metal Laser Sintering |
| DMP | Direct Metal Printing |
| EBAM | Electron Beam Additive Manufacturing |
| EBM | Electron Beam Leting |
| EBPD | Electron Beam Powder Bed |
| FDM | Fused Deposition Modeling |
| IPD | Indirect Power Bed |
| LCT | Laser Cladding Technology |
| LDT | Laser Deposition Technology |
| LDW | Laser Deposition Welding |
| LDWM | Laser Deposition Welding with integrated Milling |
| LENS | Laser Engineering Net Shape |
| LFMT | Laser Freeform Manufacturing Technology |
| LMD-p | Laser Metal Deposition-powder |
| LMD-w | Laser Metal Deposition-wire |
| LPB | Laser Powder Bed |
| LPD | Laser Puddle Deposition |
| LRT | Laser Repair Technology |
| PDED | Powder Directed Energy Deposition |
| SLA | Stereolithography |
| SLM | Selective Laser Melting |
| SLS | Selective Laser Sintering |
| SPD | Small Puddle Deposition |

Each of the components of the orthopedic implant 100 described above may represent a module, a segment, or a portion of program code, which includes one or more instructions executable by a processor or computing device for creating such devices using an additive-manufacturing system. The program code may be stored on any type of computer readable medium, for example, such as a storage device including a disk or hard drive. The computer readable medium may include non-transitory computer readable medium, for example, such as computer-readable media that stores data for short periods of time like register memory, processor cache and Random Access Memory (RAM). The computer readable medium may also include non-transitory media, such as secondary or persistent long term storage, like read only memory (ROM), optical or magnetic disks, compact-disc read only memory (CD-ROM), for example. The computer readable media may also be any other volatile or non-volatile storage systems. The computer readable medium may be considered a computer readable storage medium, for example, or a tangible storage device.

Further, curing the uncured osteostimulative material may comprise heat treating the orthopedic implant 100 after the uncured osteostimulative material is applied to the exterior surface of the orthopedic implant 100. Because high deposition temperature is needed in order to obtain high quality of MgO films, the curing temperature may be varied from 400°C to 500°C in 25°C intervals. The annealing curing is inversely proportional to the thickness of the mixture of osteostimulative material. After cooking in an oven, the orthopedic implant 100 may then be air dried. The process of heat treating will reduce drying time exponentially compared with just applying the osteostimulative material to the exterior surface of the orthopedic implant 100 and allowing it to cure without the aid of heat.

In operation, the present invention may be used in a method for securing an orthopedic implant to a bone, the method comprising: (a) providing the orthopedic implant 100 of any of the embodiments described above, (b) forming a tunnel in the bone, (c) inserting the second end of the elongated member of the orthopedic implant into the tunnel in the bone, and (d) applying an uncured osteostimulative material to one or more of the first channel and the second channel of the elongated member.

In one example, the method further includes inserting a ligament in the tunnel in the bone, and inserting the second end of the elongated member of the orthopedic implant into the tunnel in the bone such that the elongated member fills a substantial portion of the tunnel, wherein the ligament is securely fixed between the elongated member and an inner surface of the tunnel in the bone.

In another example, the method further includes positioning a bone graft in one or more of the first channel 108 and the second channel 112 of the elongated member 102.

In another example, the present invention may be used in a method for securing an orthopedic implant to a bone, the method comprising: (a) providing the orthopedic implant, (b) removing a portion of the bone to create a cavity, and (c) inserting the orthopedic implant into the cavity in the bone, wherein an entirety of the orthopedic implant comprises a cured osteostimulative material, and wherein the orthopedic implant is completely absorbed in-vivo thereby enabling natural bone structure to replace the structure of the orthopedic implant such that there is no void left behind after the orthopedic implant is absorbed in-vivo.

As discussed above, making the entirety of the orthopedic implant from a cured osteostimulative material has a number of advantages. In particular, such a material allows the orthopedic implant to be absorbed in-vivo, producing increased fixation strength and faster absorption into the body. In contrast to traditional metal alloy bone screws, there is no need to remove the orthopedic implant after a certain period of time because the material of the orthopedic implant enables bone to actually replace the structure of the orthopedic implant. As such, there is no void that is left behind after the orthopedic implant is absorbed in-vivo. Instead, the orthopedic implant is replaced with bone structure grown naturally in the body and the resulting fixation strength is very strong. As such, the orthopedic implant may be completely resorbable.

The cured osteostimulative material of the orthopedic implant may take a variety of forms, as discussed above. In particular, in one example the cured osteostimulative material comprises KH₂PO₄ in an amount between about 20-70 dry weight percent, MgO in an amount between 10-50 dry weight percent, a calcium containing compound, a poly-lactic acid, and either magnesium phosphate or potassium phosphate. In one example, the poly-lactic acid comprises one of Poly(L-lactic acid) PLA, poly(L, DL-lactide) PLDLA, and poly(L-lactide-co-glycolide) PLGA. In one example, the cured osteostimulative material further comprises a bioactive therapeutic agent. In one such example, the bioactive therapeutic agent comprises one of amikacin, butirosin, dideoxykanamycin, fortimycin, gentamycin, kanamycin, lividomycin, neomycin, netilmicin, ribostamycin, sagamycin, seldomycin and epimers thereof, sisomycin, sorbistin, spectinomycin and tobramycin. In another example, the cured osteostimulative material further comprises a sugar, and wherein the sugar comprises one of sugar alcohols, sugar acids, amino sugars, sugar polymers glycosaminoglycans, glycolipids, sugar substitutes and combinations thereof.

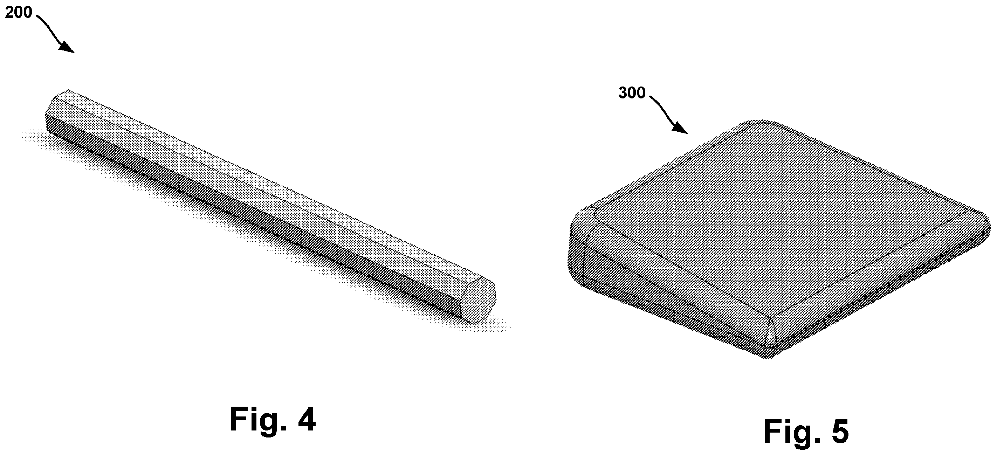

Further, the orthopedic implant may take a variety of forms. In one example, the orthopedic implant comprises the orthopedic implant 100 described above in relation to Figures 1-3. In another example, as shown in Figure 4, the orthopedic implant comprises a pin 200. In another example, as shown in Figure 5, the orthopedic implant comprises a wedge 300. Other forms of orthopedic implants are possible as well with the method described above.

While various aspects and embodiments have been disclosed herein, other aspects and embodiments will be apparent to those skilled in the art. The various aspects and embodiments disclosed herein are for purposes of illustration and are not intended to be limiting, with the true scope of the invention given by the appended claims. It is also to be understood that the terminology used herein is for the purpose of describing particular embodiments only, and is not intended to be limiting, with the scope being provided by the appended claims.

Because many modifications, variations, and changes in detail can be made to the described example, it is intended that all matters in the preceding description and shown in the accompanying figures be interpreted as illustrative and not in a limiting sense, with the scope being provided by the appended claims.

## Claims

1. An orthopedic implant (100) for fixing devices to or in a bone of a patient, comprising:
an elongated member (102) having a first end (104) and a second end (106) opposite the first end (104), wherein the elongated member (102) is tapered at the second end (106) such that a width of the second end (106) is less than a width of the first end (104);
a first channel (108) positioned on a first side (110) of the elongated member (102) and extending from the first end (104) to the second end (106);
a second channel (112) positioned on a second side (114) of the elongated member (102) and extending from the first end (104) to the second end (106); and
one or more through holes (116) connecting the first channel (108) to the second channel (112), **characterised by**
a plurality of threads on at least a portion of an exterior of the elongated member.

2. The orthopedic implant (100) of claim 1,
wherein the one or more through holes (116) comprises a plurality of through holes (116).

3. The orthopedic implant (100) of any one of claims 1-2, wherein the first channel (108) is recessed in the first side (110) of the elongated member (102), and wherein the second channel (112) is recessed in the second side (114) of the elongated member (102).

4. The orthopedic implant (100) of any one of claims 1-3, wherein the elongated member (102) comprises titanium, polyetheretherketone (PEEK), polyurethane, or bone.

5. The orthopedic implant (100) of any one of claims 1-4, wherein at least a portion of an exterior surface of the elongated member (102) includes a cured osteostimulative material.

6. The orthopedic implant (100) of claim 5, wherein the cured osteostimulative material comprises KH₂PO₄ in an amount between about 20-70 dry weight percent, MgO in an amount between 10-50 dry weight percent, a calcium containing compound, a poly-lactic acid, and either magnesium phosphate or potassium phosphate different from KH₂PO₄.

7. The orthopedic implant (100) of claim 6, wherein the poly-lactic acid comprises one of Poly(L-lactic acid) PLA, poly(L, DL-lactide) PLDLA, and poly(L-lactide-co-glycolide) PLGA.

8. The orthopedic implant (100) of any one of claims 6-7, further comprising a bioactive therapeutic agent.

9. The orthopedic implant (100) of claim 8, wherein the bioactive therapeutic agent comprises one of amikacin, butirosin, dideoxykanamycin, fortimycin, gentamycin, kanamycin, lividomycin, neomycin, netilmicin, ribostamycin, sagamycin, seldomycin and epimers thereof, sisomycin, sorbistin, spectinomycin and tobramycin.

10. The orthopedic implant (100) of any one of claims 6-9, wherein the cured osteostimulative material further comprises a sugar, and wherein the sugar comprises one of sugar alcohols, sugar acids, amino sugars, sugar polymers glycosaminoglycans, glycolipids, sugar substitutes and combinations thereof.

11. The orthopedic implant (100) of any one of claims 6-10, wherein a thickness of the cured osteostimulative material on the exterior surface of the elongated member (102) ranges from 200 µm to 50 mm.

12. The orthopedic implant (100) of any one of claims 6-11, wherein the exterior surface includes a plurality of grooves, and wherein the cured osteostimulative material is positioned in one or more of the plurality of grooves.

13. The orthopedic implant (100) of any one of claims 6-12, wherein an entirety of the elongated member (102) comprises the cured osteostimulative material.

14. The orthopedic implant (100) of any one of claims 1-13, wherein the elongated member (102) is resorbable.

## Patentansprüche

1. Orthopädisches Implantat (100) zum Befestigen von Vorrichtungen an oder in einem Knochen eines Patienten, umfassend:
ein längliches Element (102) mit einem ersten Ende (104) und einem zweiten Ende (106) abgewandt von dem ersten Ende (104), wobei das längliche Element (102) an dem zweiten Ende (106) verjüngt ist, sodass eine Breite des zweiten Endes (106) geringer als eine Breite des ersten Endes (104) ist;
einen ersten Kanal (108), der auf einer ersten Seite (110) des länglichen Elements (102) positioniert ist und sich von dem ersten Ende (104) zu dem zweiten Ende (106) erstreckt;
einen zweiten Kanal (112), der auf einer zweiten Seite (114) des länglichen Elements (102) positioniert ist und sich von dem ersten Ende (104) zu dem zweiten Ende (106) erstreckt; und
ein oder mehrere Durchgangslöcher (116), die den ersten Kanal (108) mit dem zweiten Kanal (112) verbinden, **gekennzeichnet durch**
eine Vielzahl von Gewinden an mindestens einem Abschnitt eines Äußeren des länglichen Elements.

2. Orthopädisches Implantat (100) nach Anspruch 1,
wobei das eine oder die mehreren Durchgangslöcher (116) eine Vielzahl von Durchgangslöchern (116) umfassen.

3. Orthopädisches Implantat (100) nach einem der Ansprüche 1-2, wobei der erste Kanal (108) in der ersten Seite (110) des länglichen Elements (102) vertieft ist und wobei der zweite Kanal (112) in der zweiten Seite (114) des länglichen Elements (102) vertieft ist.

4. Orthopädisches Implantat (100) nach einem der Ansprüche 1-3, wobei das längliche Element (102) Titan, Polyetheretherketon (PEEK), Polyurethan oder Knochen umfasst.

5. Orthopädisches Implantat (100) nach einem der Ansprüche 1-4, wobei mindestens ein Abschnitt einer äußeren Oberfläche des länglichen Elements (102) ein gehärtetes osteostimulierendes Material beinhaltet.

6. Orthopädisches Implantat (100) nach Anspruch 5, wobei das gehärtete osteostimulierende Material KH₂PO₄ in einer Menge zwischen etwa 20-70 Trockengewichtsprozent, MgO in einer Menge zwischen 10-50 Trockengewichtsprozent, eine Calcium enthaltende Verbindung, eine Polymilchsäure und entweder Magnesiumphosphat oder Kaliumphosphat, das sich von KH₂PO₄ unterscheidet, umfasst.

7. Orthopädisches Implantat (100) nach Anspruch 6, wobei die Polymilchsäure eines von Poly(L-milchsäure) PLA, Poly(L, DL-lactid) PLDLA und Poly(L-lactid-co-glycolid) PLGA umfasst.

8. Orthopädisches Implantat (100) nach einem der Ansprüche 6-7, ferner umfassend ein bioaktives therapeutisches Mittel.

9. Orthopädisches Implantat (100) nach Anspruch 8, wobei das bioaktive therapeutische Mittel eines von Amikacin, Butirosin, Dideoxykanamycin, Fortimycin, Gentamycin, Kanamycin, Lividomycin, Neomycin, Netilmicin, Ribostamycin, Sagamycin, Seldomycin und Epimeren davon, Sisomycin, Sorbistin, Spectinomycin und Tobramycin umfasst.

10. Orthopädisches Implantat (100) nach einem der Ansprüche 6-9, wobei das gehärtete osteostimulierende Material ferner einen Zucker umfasst und wobei der Zucker eines von Zuckeralkoholen, Zuckersäuren, Aminozuckern, Zuckerpolymeren Glycosaminoglycane, Glycolipiden, Zuckeraustauschstoffen und Kombinationen davon umfasst.

11. Orthopädisches Implantat (100) nach einem der Ansprüche 6-10, wobei eine Dicke des gehärteten osteostimulierenden Materials auf der äußeren Oberfläche des länglichen Elements (102) im Bereich von 200 µm bis 50 mm liegt.

12. Orthopädisches Implantat (100) nach einem der Ansprüche 6-11, wobei die äußere Oberfläche eine Vielzahl von Rillen beinhaltet und wobei das gehärtete osteostimulierende Material in einer oder mehreren der Vielzahl von Rillen positioniert ist.

13. Orthopädisches Implantat (100) nach einem der Ansprüche 6-12, wobei eine Gesamtheit des länglichen Elements (102) das gehärtete osteostimulierende Material umfasst.

14. Orthopädisches Implantat (100) nach einem der Ansprüche 1-13, wobei das längliche Element (102) resorbierbar ist.

## Revendications

1. Implant orthopédique (100) destiné à fixer des dispositifs sur un os d'un patient ou dans celui-ci, comprenant :
un élément allongé (102) possédant une première extrémité (104) et une seconde extrémité (106) opposée à la première extrémité (104), ledit élément allongé (102) étant conique au niveau de la seconde extrémité (106) de sorte qu'une largeur de la seconde extrémité (106) soit inférieure à une largeur de la première extrémité (104) ;
un premier canal (108) positionné sur un premier côté (110) de l'élément allongé (102) et s'étendant de la première extrémité (104) à la seconde extrémité (106) ;
un second canal (112) positionné sur un second côté (114) de l'élément allongé (102) et s'étendant de la première extrémité (104) à la seconde extrémité (106) ; et
un ou plusieurs trous traversants (116) reliant le premier canal (108) au second canal (112), **caractérisés par** une pluralité de filets sur au moins une partie d'un extérieur de l'élément allongé.

2. Implant orthopédique (100) selon la revendication 1,
lesdits un ou plusieurs trous traversants (116) comprenant une pluralité de trous traversants (116).

3. Implant orthopédique (100) selon l'une quelconque des revendications 1 à 2, ledit premier canal (108) étant en retrait dans le premier côté (110) de l'élément allongé (102), et ledit second canal (112) étant en retrait dans le second côté (114) de l'élément allongé (102).

4. Implant orthopédique (100) selon l'une quelconque des revendications 1 à 3, ledit élément allongé (102) comprenant du titane, de la polyétheréthercétone (PEEK), du polyuréthane ou de l'os.

5. Implant orthopédique (100) selon l'une quelconque des revendications 1 à 4, au moins une partie d'une surface extérieure de l'élément allongé (102) comprenant un matériau ostéostimulant durci.

6. Implant orthopédique (100) selon la revendication 5, ledit matériau ostéostimulant durci comprenant du KH₂PO₄ en une quantité comprise entre environ 20 et 70 pour cent en poids sec, du MgO en une quantité comprise entre 10 et 50 pour cent en poids sec, un composé contenant du calcium, un acide poly-lactique et soit du phosphate de magnésium soit du phosphate de potassium différent du KH₂PO₄.

7. Implant orthopédique (100) selon la revendication 6, ledit acide poly-lactique comprenant un composé parmi le poly(acide L-lactique) PLA, le poly(L, DL-lactide) PLDLA et le poly(L-lactide-co-glycolide) PLGA.

8. Implant orthopédique (100) selon l'une quelconque des revendications 6 à 7, comprenant en outre un agent thérapeutique bioactif.

9. Implant orthopédique (100) selon la revendication 8, ledit agent thérapeutique bioactif comprenant un parmi l'amikacine, la butirosine, la didésoxykanamycine, la fortimycine, la gentamycine, la kanamycine, la lividomycine, la néomycine, la nétilmicine, la ribostamycine, la sagamycine, la seldomycine et leurs épimères, la sisomycine, la sorbistine, la spectinomycine et la tobramycine.

10. Implant orthopédique (100) selon l'une quelconque des revendications 6 à 9, ledit matériau ostéostimulant durci comprenant en outre un sucre, et ledit sucre comprenant un parmi les alcools de sucre, les acides de sucre, les sucres aminés, les glycosaminoglycanes de polymères de sucre, des glycolipides, les substituts de sucre et leurs combinaisons.

11. Implant orthopédique (100) selon l'une quelconque des revendications 6 à 10, une épaisseur du matériau ostéostimulant durci sur la surface extérieure de l'élément allongé (102) étant comprise entre 200 µm et 50 mm.

12. Implant orthopédique (100) selon l'une quelconque des revendications 6 à 11, ladite surface extérieure comprenant une pluralité de rainures, et ledit matériau ostéostimulant durci étant positionné dans une ou plusieurs rainures de la pluralité de rainures.

13. Implant orthopédique (100) selon l'une quelconque des revendications 6 à 12, une totalité de l'élément allongé (102) comprenant le matériau ostéostimulant durci.

14. Implant orthopédique (100) selon l'une quelconque des revendications 1 à 13, ledit élément allongé (102) étant résorbable.
